(19) Europäisches Patentamt
European Patent Office
Office européen des brevets

(11) Veröffentlichungsnummer: **0 033 476**
**B1**

# (12) EUROPÄISCHE PATENTSCHRIFT

(45) Veröffentlichungstag der Patentschrift: **21.03.84**

(51) Int. Cl.³: **C 07 D253/06**, C 07 C103/12, C 07 C102/08

(21) Anmeldenummer: **81100405.0**

(22) Anmeldetag: **21.01.81**

## (54) Verfahren zur Herstellung von 4-Amino-6-tert.-butyl-3-methylthio-1,2,4-triazin-5(4H)-on.

(30) Priorität: **31.01.80 DE 3003541**

(43) Veröffentlichungstag der Anmeldung: **12.08.81 Patentblatt 81/32**

(45) Bekanntmachung des Hinweises auf die Patenterteilung: **21.03.84 Patentblatt 84/12**

(84) Benannte Vertragsstaaten: **BE CH DE FR GB IT LI NL**

(56) Entgegenhaltungen:
**DE-A- 2 708 185**
**DE-A- 2 726 016**
**DE-A- 2 733 180**
**FR-A- 1 547 854**
**CHEMISCHE BERICHTE, Band 97, 1964, WEINHEIM (DE) A. DORNOW et al.: "Über 1,2,4-triazine I", Seiten 2173-2179**
**JOURNAL OF THE AMERICAN CHEMICAL SOCIETY, Band 73, 1951, COLUMBUS, OHIO (US) R.C. THOMAS et al.: "The preparation of alpha C-labeled pyruvic acid and a study of the hydrolysis of pyruvonitrile", Seite 5914**
**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Schmidt, Thomas, Dr.**
**Am Bandenfeld 72**
**D-5657 Haan 1 (DE)**
Erfinder: **Wittig, Andreas, Dr.**
**Am Friedenshain 32**
**D-5600 Wuppertal 1 (DE)**
Erfinder: **Sehnem, Hans-Peter**
**Nüllerstrasse 90**
**D-5600 Wuppertal (DE)**
Erfinder: **Krätzer, Hans, Dr.**
**Am Acker 17**
**D-5600 Wuppertal 1 (DE)**
Erfinder: **Singer, Rolf Jürgen, Dr.**
**Platzhoffstrasse 55**
**D-5600 Wuppertal 1 (DE)**

**EP 0 033 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 4-Amino-6-tert.-butyl-3-methylthio-1,2,4-triazin-5(4H)-on

Die vorliegende Erfindung betrifft ein Verfahren zur Herstellung des bekannten, herbizid wirksamen 4-Amino-6-tert.-butyl-3-methylthio-1,2,4-triazin-5(4H)-ons.

Es ist bereits bekannt geworden, daß man das 4-Amino-6-tert.-butyl-3-methylthio-1,2,4-triazin-5(4H)-on (V) erhält, wenn man in einer ersten Stufe Pivaloylcyanid der Formel

$$(CH_3)_3C—CO—CN \qquad (I)$$

mit tert.-Butanol oder Isobutylen in Gegenwart einer starken Säure, wie insbesondere Schwefelsäure, gegebenenfalls in Gegenwart eines organischen Lösungsmittels, wie insbesondere Eisessig, Dichlormethan oder höherer Ether, bei Temperaturen zwischen – 20 bis + 50 °C zum Trimethylbrenztraubensäure-N-tert.-butylamid der Formel

$$(CH_3)_3C—CO—CO—NH—C(CH_3)_3 \qquad (II)$$

umsetzt, dieses in einer zweiten Stufe, gegebenenfalls nach vorheriger Verseifung zur freien Trimethylbrenztraubensäure der Formel

$$(CH_3)_3C—CO—COOH \qquad (III)$$

mit Thiocarbohydrazid ($H_2N—NH—CS—NH—NH_2$) zum 4-Amino-6-tert.-butyl-3-mercapto-1,2,4-triazin-5(4H)-on der Formel

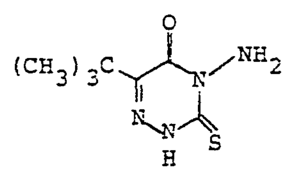

(IV)

kondensiert und dieses in einer dritten Stufe in üblicher Weise mit einem Methylierungsmittel, wie Methyljodid oder -bromid, zum 4-Amino-6-tert.-butyl-3-methylthio-1,2,4-triazin-5(4H)-on der Formel

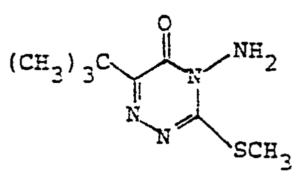

(V)

umsetzt (vgl. DE-OS 27 33 180).

Dieses Verfahren, dessen erste Stufe unter den Bedingungen der sogenannten Ritter-Reaktion verläuft, hat jedoch den Nachteil, daß man als Hilfsstoff Isobutylen oder tert.-Butanol, zweckmäßigerweise in überstöchiometrischen Mengen, einsetzen muß.

Die Umsetzung des α-Ketosäureamids (II) mit Thiocarbohydrazid zu (IV) gemäß der zweiten Verfahrensstufe ist in grundsätzlicher Form vorbekannt (vgl. DE-OS 21 65 554). Die Umsetzung der freien α-Ketosäure (III) mit Thiocarbohydrazid zu (IV) ist vorbeschrieben (vgl. z. B. DE-OS 24 60 889). Die dritte Verfahrensstufe ist gleichfalls vorbeschrieben (vgl. z. B. DE-OS 20 03 144, DE-OS 27 29 761 ; US-PS 3 897 429).

Es wurde nun gefunden, daß man das bekannte 4-Amino-6-tert.-butyl-3-methylthio-1,2,4-triazin-5(4H)-on (Metribuzin) der Formel (V)

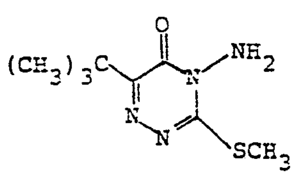

(V)

ausgehend von Pivaloylcyanid (I) in einfacher Weise mit hoher Ausbeute herstellen kann, wenn man in einer ersten Stufe das Pivaloylcyanid in Gegenwart einer unter den Reaktionsbedingungen flüssigen Carbonsäure mit 1-6 C-Atomen als Lösungsmittel im Druckbereich von 1-10 bar zuerst mit wasserfreiem Halogenwasserstoff und gegebenenfalls danach mit Wasser jeweils bei Temperaturen zwischen – 50 und

+ 50 °C umsetzt und das hierbei gebildete, bisher nicht bekannte Trimethylbrenztraubensäureamid der Formel (VI)

$$(CH_3)_3C—CO—CO—NH_2 \qquad (VI)$$

in einer zweiten Stufe in an sich bekannter Weise entweder direkt in der anfallenden Lösung oder nach Zwischenisolierung, gegebenenfalls nach vorheriger Verseifung zur freien Trimethylbrenztraubensäure (III), mit Thiocarbohydrazid ($H_2N—NH—CS—NH—NH_2$) in halogenwasserstoffsaurer, wäßriger Lösung bei Temperaturen von 20 bis 100 °C zu 4-Amino-6-tert.-butyl-3-mercapto-1,2,4-triazin-5(4H)-on (IV) umsetzt und dieses in einer dritten Stufe in bekannter Weise in alkalischer Lösung mittels Methyljodid oder Methylbromid methyliert.

Es war nach dem Stand der Technik bisher nicht möglich, Pivaloylcyanid über Trimethylbrenztraubensäureamid (VI) zu Trimethylbrenztraubensäure (III) zu hydrolysieren.

Bekanntlich ist ganz allgemein die Herstellung von α-Ketocarbonsäuren bzw. deren Amiden durch Verseifung von entsprechenden Acylcyaniden mit erheblichen Schwierigkeiten verbunden, weil es praktisch nicht gelingt, unter den Hydrolysebedingungen eine Spaltung der Acylcyanide in Blausäure und 1 C-Atom weniger als das eingesetzte Acylcyanid enthaltende Carbonsäuren ganz zu unterdrücken (vgl. Angew. Chem. *68*, S. 430 (1956)).

Unter speziellen Verfahrensbedingungen gelingt es jedoch, jeweils mit guten Ausbeuten Brenztraubensäurenitril ($CH_3—CO—CN$) zu Brenztraubensäureamid ($CH_3—CO—CO—NH_2$) sowie bestimmte C-substituierte Brenztraubensäurenitrile der allgemeinen Formel $R^1R^2R^3C—CO—CN$ (worin $R^1$, $R^2$ und $R^3$ mit bestimmten Einschränkungen für Wasserstoff und gegebenenfalls substituierte Alkyl- und Cycloalkylreste stehen) zu den entsprechenden Amiden der Formel $R^1R^2R^3C—CO—CO—NH_2$ zu hydrolysieren (vgl. J. Amer. Chem. Soc. *73*, S. 5914 (1951) bzw. DE-A1 2 708 184 und DE-A1 2 708 185). Hierzu werden die α-Ketocarbonsäurenitrile (= Acylcyanide) in etherischer Lösung zunächst mit trockenem gasförmigem Chlorwasserstoff und anschließend mit Wasser behandelt ; die gebildeten α-Ketocarbonsäureamide werden in üblicher Weise isoliert.

Nach diesen aus dem Stand der Technik bekannten Verfahren gelingt es dagegen nicht, wie eigene Versuche gezeigt haben, in entsprechender Weise Trimethylbrenztraubensäurenitril, d. h. Pivaloylcyanid (I), mit nennenswerter Ausbeute zu Trimethylbrenztraubensäureamid (VI) zu hydrolysieren. Insofern ist es als überraschend zu bereichnen, daß es unter den Bedingungen der ersten Stufe des erfindungsgemäßen Verfahrens gelingt, Trimethylbrenztraubensäureamid (VI) in guter Ausbeute zu erhalten, wodurch der neue und vereinfachte Syntheseweg von Pivaloylcyanid zu dem Herbizid Metribuzin ermöglicht wird.

Insbesondere war aufgrund des Standes der Technik nicht zu erwarten, daß Stufe 1 des erfindungsgemäßen Verfahrens in Gegenwart einer niederaliphatischen Carbonsäure als Lösungsmittel in guten Ausbeuten verläuft, zumal diese organischen Säuren unter den Reaktionsbedingungen, insbesondere gegenüber Halogenwasserstoffen, nicht als inert anzusehen sind.

Das erfindungsgemäße Verfahren vermeidet die zuvor erwähnten, mit dem chemisch ähnlichsten Verfahren zur Herstellung des herbiziden Wirkstoffes (V) verbundenen Nachteile (vgl. DE-OS 27 33 180) ; dies bedeutet eine wesentliche technische Vereinfachung.

Gegenüber anderen vorbekannten Verfahren (vgl. z. B. DE-OS' en 20 03 144 ; 21 65 554 ; 24 60 889 ; 24 60 909 ; 26 48 300) zur Herstellung des Wirkstoffes (V) aus anderen Pivalinsäurederivaten bzw. Pinakolin hat das erfindungsgemäße Verfahren ebenfalls den technischen Vorteil starker Vereinfachung. Gegenüber den von Pinakolin ausgehenden Verfahren ist ein zusätzlicher Vorteil in der unterschiedlichen Rohstoffbasis zu sehen.

Verwendet man in der ersten Verfahrensstufe Eisessig als Lösungsmittel, Bromwasserstoff als Halogenwasserstoff, setzt in der zweiten Stufe das Amid-Zwischenprodukt direkt als solches mit Thiocarbohydrazid um und verwendet in der dritten Stufe Methylbromid als Methylierungsmittel, so kann der Reaktionsverlauf zusammenfassend nochmals durch das folgende Formelschema wiedergegeben werden :

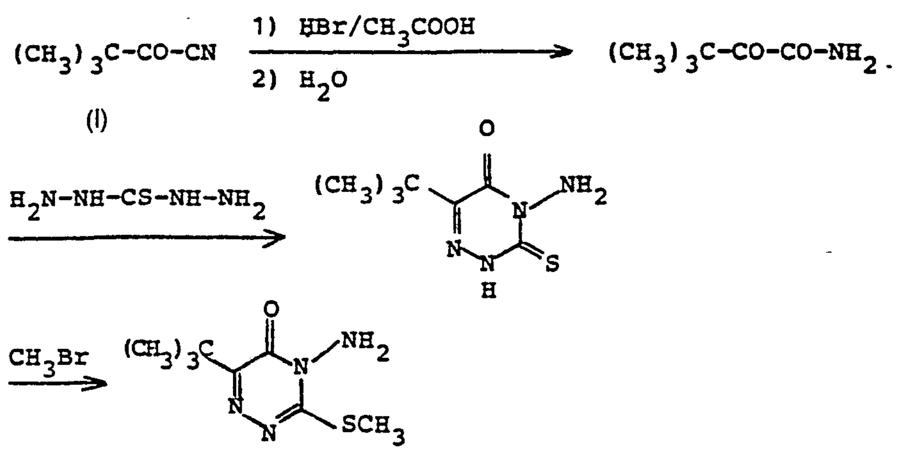


(I) (VI)

(IV)

(V)

Das als Ausgangsprodukt verwendete Pivaloylcyanid (I) ist bekannt und kann z. B. durch Umsetzung von Pivaloylchlorid mit Kupfer-(I)-cyanid hergestellt werden (vgl. z. B. J. Amer. Chem. Soc. *72*, S. 2793 (1950)).

Zu Stufe 1 des erfindungsgemäßen Verfahrens seien im einzelnen noch folgende Angaben gemacht :

Die Reaktionstemperaturen können bei Durchführung dieser Verfahrensstufe in einem größeren Bereich variiert werden. Im allgemeinen arbeitet man, wie oben angegeben, zwischen − 50 und + 50 °C, vorzugsweise bei − 20 bis + 30 °C.

Die erste Verfahrensstufe kann unter Normaldruck, aber auch unter erhöhtem Druck, vorzugsweise im Druckbereich von 1 bis 10 bar, durchgeführt werden.

Die erste Verfahrensstufe wird mit Hilfe von wasserfreiem Halogenwasserstoff durchgeführt. Vorzugsweise wird hierfür trockener Chlorwasserstoff oder bromwasserstoff verwendet.

Diese Verfahrensstufe wird in Gegenwart einer unter den Reaktionsbedingungen flüssigen Carbonsäure mit 1-6 C-Atomen als Lösungsmittel durchgeführt. Als solche Carbonsäuren kommen insbesondere wasserfreie Essigsäure, Propionsäure und Ameisensäure in Frage. Besonders bevorzugt ist Eisessig.

Ein Zusatz von Wasser zum Reaktionsgemisch zur Bildung des Amids (VI) in der ersten Verfahrensstufe ist nicht zwingend erforderlich : durch Einwirkung des Halogenwasserstoffs auf die Carbonsäure bildet sich neben einer gewissen Menge Carbonsäurehalogenid die entsprechende Menge Wasser, welche zur Hydrolyse des eingesetzten Pivaloylcyanids bzw. möglicher intermediärer Zwischenstufen ausreichend sein kann. In solchen Fällen fungiert die eingesetzte Carbonsäure nicht nur als Lösungsmittel, sondern zum Teil auch als Reaktionspartner (wobei allerdings auch alternative Reaktionsmechanismen denkbar sind). Jedenfalls ist es bei dieser Ausführungsform der ersten Verfahrensstufe nicht erforderlich, stöchiometrische Mengen an Wasser einzusetzen.

Bei der Durchführung der ersten Stufe des erfindungsgemäßen Verfahrens setzt man auf 1 Mol Pivaloylcyanid (I) im allgemeinen 1 bis 10 Mol an Halogenwasserstoff und 0 bis 10 Mol Wasser, vorzugsweise 1 bis 8 Mol an Halogenwasserstoff und 0 bis 2,5 Mol Wasser ein.

Das in dieser Reaktionsstufe gebildete, neue Trimethylbrenztraubensäureamid (VI) kann, wenn gewünscht, in üblicher Weise zwischenisoliert werden.

Die zweite Stufe des erfindungsgemäßen Verfahrens erfolgt vorzugsweise ohne vorherige Zwischenisolierung des Trimethylbrenztraubensäureamids (VI) und ohne Verseifung desselben zur freien Säure (III).

Die Umsetzung wird in Gegenwart einer halogenwasserstoffsauren, vorzugsweise einer salzsauren, wäßrigen Lösung durchgeführt.

Die Reaktionstemperaturen können bei dieser Verfahrensstufe in einem größeren Bereich variiert werden. Im allgemeinen arbeitet man zwischen 20 und 100 °C, vorzugsweise zwischen 50 und 90 °C.

Bei der Durchführung der zweiten Verfahrensstufe setzt man die Ausgangsstoffe vorzugsweise in molaren Mengen ein. Die Isolierung des Zwischenproduktes (IV) erfolgt in üblicher Weise.

Die dritte Stufe des erfindungsgemäßen Verfahrens erfolgt in bekannter Weise durch Umsetzung mit einem Methylhalogenid, wie Methylbromid oder Methyljodid, in Gegenwart einer Base, wie Natriumhydroxid, in wäßriger Lösung bei Temperaturen zwischen 0 und 50 °C.

Das erfindungsgemäß herstellbare 4-Amino-6-tert.-butyl-3-methylthio-1,2,4-triazin-5(4H)-on der Formel (V) zeichnet sich bekanntermaßen durch sehr gute herbizide Wirksamkeit aus (vgl. z. B. DE-PS 17 95 784).

Die nachfolgenden Herstellungsbeispiele sollen das erfindungsgemäße Verfahren näher illustrieren.

## Herstellungsbeispiele

### Beispiel 1

Zu 800 g einer Lösung von Bromwasserstoff in Eisessig (36 %ig) werden unter Rühren 111 g (1 Mol) Pivaloylcyanid bei Raumtemperatur zugetropft. Nach beendeter Zugabe rührt man drei Stunden bei Raumtemperatur nach. Anschließend tropft man 9 ml (0,5 Mol) Wasser bei 20 bis 25 °C zu und läßt eine Stunde bei Raumtemperatur nachrühren. Danach werden der Eisessig und der überschüssige Bromwasserstoff im Vakuum abgezogen, und das anfallende Trimethylbrenztraubensäureamid wird in eine Mischung von 107,6 g Thiocarbohydrazid, 420 g Wasser und 140,6 g konzentrierte Salzsäure eingetragen. Dieses Reaktionsgemisch rührt man 1,5 Stunden bei 90 °C und 12 Stunden bei Raumtemperatur nach. Danach wird auf 0 °C abgekühlt und das ausfallende kristallisat abfiltriert, mit 200 ml Wasser gewaschen und bei 100 °C im Vakuum getrocknet. Man erhält 168,6 g (84,3 % der Theorie) 4-Amino-6-tert.-butyl-3-mercapto-1,2,4-triazin-5(4H)-on (IV) vom Schmelzpunkt 205-209 °C.

In eine Mischung von 820 g 45 %iger Natronlauge und 550 g Wasser werden unter Rühren 168,6 g (0,84 Mol) 4-Amino-6-tert.-butyl-3-mercapto-1,2,4-triazin-5(4H)-on eingetragen. Nach vollständiger Lösung des Produktes werden 140 g Methyljodid so zugegeben, daß die Innentemperatur 30 °C nicht übersteigt. Nach beendeter Zugabe wird die Reaktionslösung noch zwei Stunden bei Raumtemperatur

gerührt. Danach wird der entstandene Feststoff abgesaugt, mit 1 000 ml Wasser gewaschen und im Vakuumtrockenschrank bei 60 °C getrocknet. — Man erhält 146 g (81 % der Theorie) 4-Amino-6-tert.-butyl-3-methylthio-1,2,4-triazin-5(4H)-on (V) vom Schmelzpunkt 123-125 °C.

Beispiel 2

In eine Mischung von 222 g (2 Mol) Pivaloylcyanid und 120 g (2 Mol) Eisessig wird bei 8 bar und 20 bis 25 °C vier Stunden lang Chlorwasserstoffgas eingeleitet. Danach wird entspannt und überschüssiger Eisessig im Vakuum abgezogen. Danach wird das Produkt in eine Mischung von 212 g (2 Mol) Thiocarbohydrazid, 840 g Wasser und 280 g konzentrierte Salzsäure gegeben. Nach beendeter Zugabe erwärmt man die Reaktionsmischung 1,5 Stunden lang auf 90 °C und läßt dann über Nacht bei Raumtemperatur nachrühren. Anschließend wird auf 0 °C abgekühlt, der anfallende Feststoff abgesaugt, mit 400 ml kaltem Wasser gewaschen und im Vakuum bei 40 °C getrocknet. — Man erhält 314,4 g (78,6 % der Theorie) 4-Amino-6-tert.-butyl-3-mercapto-1,2,4-triazin-5(4H)-on (IV) vom Schmelzpunkt 205-209 °C.

Die Methylierung von (IV) zu (V) erfolgt entsprechend beispiel 1.

**Ansprüche**

1. Verfahren zur Herstellung von 4-Amino-6-tert.-butyl-3-methylthio-1,2,4-triazin-5(4H)-on der Formel

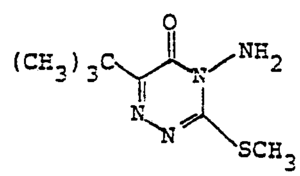

(V)

dadurch gekennzeichnet, daß man

(1) Pivaloylcyanid der Formel

$$(CH_3)_3C—CO—CN \qquad (I)$$

in Gegenwart einer unter den Reaktionsbedingungen flüssigen Carbonsäure mit 1-6 C-Atomen als Lösungsmittel im Druckbereich von 1-10 bar zuerst mit wasserfreiem Halogenwasserstoff und gegebenenfalls danach mit Wasser jeweils bei Temperaturen zwischen − 50 und + 50 °C umsetzt,

(2) das hierbei gebildete Trimethylbrenztraubensäureamid der Formel

$$(CH_3)_3C—CO—CO—NH_2 \qquad (VI)$$

in an sich bekannter Weise entweder direkt in der anfallenden Lösung oder nach Zwischenisolierung, gegebenenfalss nach vorheriger Verseifung zur freien Trimethylbrenztraubensäure, mit Thiocarbohydrazid ($H_2N—NH—CS—NH—NH_2$) in halogenwasserstoffsaurer, wäßriger Lösung bei Temperaturen von 20 bis 100 °C zu 4-Amino-6-tert.-butyl-3-mercapto-1,2,4-triazin-5(4H)-on (IV) umsetzt und

(3) dieses Zwischenprodukt (IV) in alkalischer Lösung mittels Methyljodid oder Methylbromid in üblicher Weise methyliert.

2. Verfahren nach Anspruch 1, dadurch gekennzeichnet, daß man die Umsetzung gemäß Stufe (1) bei Temperaturen zwischen − 20 und + 30 °C durchführt.

3. Verfahren nach Anspruch 1, dadurch gekennzeichnet, daß man bei der Umsetzung gemäß Stufe (1) auf 1 Mol Pivaloylcyanid 1 bis 10 Mol Halogenwasserstoff und 0 bis 10 Mol Wasser einsetzt.

4. Verfahren nach Anspruch 3, dadurch gekennzeichnet, daß man bei der Umsetzung gemäß Stufe (1) auf 1 Mol Pivaloylcyanid 1 bis 8 Mol Halogenwasserstoff und 0 bis 2,5 Mol Wasser einsetzt.

5. Verfahren nach Anspruch 1, dadurch gekennzeichnet, daß man bei der Umsetzung gemäß Stufe (1) als Carbonsäure Eisessig und als Halogenwasserstoff Chlorwasserstoff oder Bromwasserstoff einsetzt.

6. Verfahren nach Anspruch 1, dadurch gekennzeichnet, daß man das als Zwischenprodukt gebildete Trimethylbrenztraubensäureamid (VI) direkt, ohne Zwischenisolierung, weiter umsetzt.

**Claims**

1. Process for the preparation of 4-amino-6-tert.-butyl-3-methylthio-1,2,4-triazin-5(4H)-one of the formula

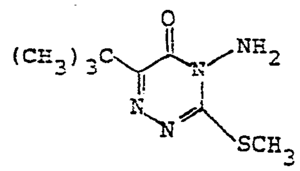

(V)

characterised in that

(1) pivaloyl cyanide of the formula

$(CH_3)_3C—CO—CN$ (I)

is first reacted with an anhydrous hydrogen halide in the presence of a carboxylic acid with 1-6 C-atoms which is liquid under the reaction conditions, as the solvent, in a pressure range of 1-10 bars, and if appropriate then with water, in each case at temperatures between − 50 and + 50 °C,

(2) the trimethylpyruvic acid amide of the formula

$(CH_3)_3C—CO—CO—NH_2$ (VI)

which is thereby formed is reacted, either directly in the solution obtained or after intermediate isolation, and if appropriate after prior saponification to give free trimethylpyruvic acid, with thiocarbohydrazide ($H_2N—NH—CS—NH—NH_2$) in an aqueous solution containing a hydrogen halide acide, at temperatures from 20 to 100 °C, in a manner which is in itself known to give 4-amino-6-tert.-butyl-3-mercapto-1,2,4-triazin-5(4H)-one (IV) and

(3) this intermediate product (IV) is methylated in alkaline solution in the customary manner by means of methyl iodide or methyl bromide.

2. Process according to Claim 1, characterised in that the reaction in stage (1) is carried out at temperatures between − 20 and + 30 °C.

3. Process according to Claim 1, characterised in that 1 to 10 mols of hydrogen halide and 0 to 10 mols of water are employed per mol of pivaloyl cyanide in the reaction in stage (1).

4. Process according to Claim 3, characterised in that 1 to 8 mols of hydrogen halide and 0 to 2.5 mols of water are employed per mol of pivaloyl cyanide in the reaction in stage (1).

5. Process according to Claim 1, characterised in that glacial acetic acid is employed as the carboxylic acid and hydrogen chloride or hydrogen bromide is employed as the hydrogen halide in the reaction in stage (1).

6. Process according to Claim 1, characterised in that the trimethylpyruvic acid amide (VI) formed as an intermediate product is further reacted directly, without intermediate isolation.

**Revendications**

1. Procédé pour la fabrication de 4-amino-6-tert.-butyl-3-méthylthio-1,2,4-triazine-5(4H) one de formule

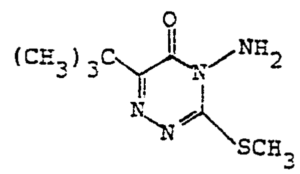

(V)

caractérisé en ce que

(1) on fait réagir le cyanure de pivaloyle de formule

$(CH_3)_3C—CO—CN$ (I)

d'abord avec l'halogénure d'hydrogène anhydre et ensuite éventuellement avec l'eau, chaque fois à des températures comprises entre − 50 et + 50 °C, en présence d'un acide carboxylique en $C_1$-$C_6$ liquide dans les conditions de réaction comme solvant, sous des pressions de 1-10 bar,

(2) on fait réagir le triméthylpyruvamide de formule

$(CH_3)_3C—CO—CO—NH_2$ (VI)

ainsi formé, de manière connue soit directement dans la solution qui se forme, soit après isolement intermédiaire, éventuellement après saponification préalable en l'acide pyruvique libre, avec le thiocarbohydrazide ($H_2N—NH—CS—NH—NH_2$) en solution aqueuse halohydrique à des températures de 20 à 100 °C en 4-amino-6-tert.-butyl-3-mercapto-1,2,4-triazine-5(4H)-one (IV) et

(3) on méthyle ce produit intermédiaire (IV) par l'iodure de méthyle ou le bromure de méthyle de manière habituelle en solution alcaline.

2. Procédé selon la revendication 1, caractérisé en ce que l'on effectue la réaction selon l'étape (1) à des températures comprises entre − 20 et + 30 °C.

3. Procédé selon la revendication 1, caractérisé en ce que, dans la réaction selon l'étape (1), on utilise 1 à 10 moles d'halogénure d'hydrogène et 0 à 10 moles d'halogénure d'hydrogène et 0 à 10 moles d'eau pour 1 mole de cyanure de pivaloyle.

4. Procédé selon la revendication 3, caractérisé en ce que, dans la réaction selon l'étape (1), on utilise 1 à 8 moles d'halogénure d'hydrogène et 0 à 2,5 moles d'eau pour 1 mole de cyanure de pivaloyle.

5. Procédé selon la revendication 1, caractérisé en ce que, dans la réaction selon l'étape (1), on utilise l'acide acétique glacial comme acide carboxylique et le chlorure d'hydrogène ou le bromure d'hydrogène comme halogénure d'hydrogène.

6. Procédé selon la revendication 1, caractérisé en ce que l'on fait à nouveau réagir directement le triméthylpyruvamide (VI) formé comme intermédiaire, sans isolement intermédiaire.